(19) Europäisches Patentamt
European Patent Office
Office européen des brevets

(11) Publication number: **0 141 267**
**B1**

(12) # EUROPEAN PATENT SPECIFICATION

(45) Date of publication of patent specification: **05.08.87**

(51) Int. Cl.$^4$: **C 07 C 53/128** // A61K31/20

(21) Application number: **84111700.5**

(22) Date of filing: **01.10.84**

(54) **Acid salts of valproic acid.**

(30) Priority: **26.10.83 US 545720**

(43) Date of publication of application:
**15.05.85 Bulletin 85/20**

(45) Publication of the grant of the patent:
**05.08.87 Bulletin 87/32**

(84) Designated Contracting States:
**BE CH DE FR GB IT LI NL SE**

(56) References cited:
**WO-A-81/00562**

(73) Proprietor: **ABBOTT LABORATORIES**
**14th Street and Sheridan Road North St**
**North Chicago Illinois 60064 (US)**

(72) Inventor: **Bauer, John Francis**
**1223 Colgate Street**
**Wilmette, IL (US)**
Inventor: **Shada, Douglas Marcel**
**7530 104th Street**
**Kenosha, WI (US)**

(74) Representative: **Modiano, Guido et al**
**MODIANO, JOSIF, PISANTY & STAUB Modiano & Associati Via Meravigli, 16**
**I-20123 Milan (IT)**

EP 0 141 267 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to valproate salts, and more particularly to certain alkali metal salts of valproic acid having improved stability.

Valproic acid or 2-propylpentanoic acid has come into wide-spread use in the treatment of epileptic seizures or convulsions. Most commonly used are valproic acid and its sodium salt; the acid is a liquid while the sodium salt is a hygroscopic solid characterized by poor stability. As a result, both have limited utility in the preparation of oral dosage forms.

Significant improvements in the forms of valproic acid-salts have been achieved in parallel studies of the Applicant.

It has been thus found that highly stable, non-hygroscopic compounds derived from valproic acid can be prepared from equimolar amounts of (a) valproic acid or diethylacetic acid and (b) a valproate salt of sodium or calcium.

It has been discovered that some of the compounds thus produced are in the nature of an oligomer. For example, the compound formed from valproic acid and sodium valproate has the structure:

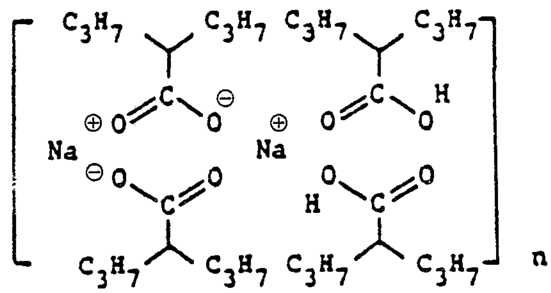

(I)

The polymer contains about 2 to 20, preferably from about 4 to 12 and most preferably about 8 of the repeating units.

A comparison of infrared spectra and X-ray diffraction patterns of sodium valproate, valproic acid and the sodium complex reveal that the above complex (I) is a chemical entity and not a mixture of the two precursors. Without limitation as to theory, the outer shell of electrons of the sodium atom is filled by coordination to the oxygen atoms of both valproic acid and valproate ions, resulting in a stable complex in which the sodium ion is completely surrounded by oxygen.

The calcium salt formed when use is made of a 2:1 mole ratio of valproic acid and valproate salt is a monomeric salt which has the structure:

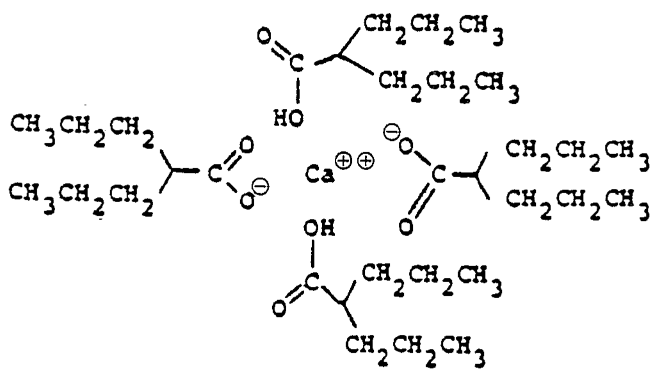

(II)

Thus, as it appears from the formula II, two moles of valproic acid and two moles of valproate ions are coordinately bonded to one calcium atom.

It has now been found that an oligomeric calcium salt of valproic acid can be formed with four moles of valproic acid complexed with a calcium ion. Without limiting the invention as to theory, it is believed that the oligomeric salt has the structure:

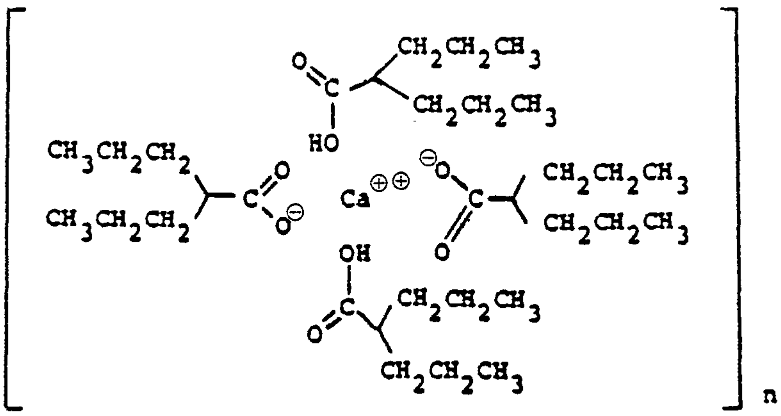

(III)

wherein n is 2 to 20.

Thus, unlike the monomeric complex formed by the association of two moles of valproic acid and one mole of calcium valproate, the salt of the present invention is oligomeric.

The compound of the present invention is a distinct chemical entity, crystalline in nature and has well defined physical and chemical properties.

The calcium salt of the present invention can be prepared by reacting one mole of calcium hydroxide with four moles of valproic acid in aqueous media, and then removing the water to recover the crystalline complex thus produced. As will be appreciated by those skilled, the inclusion of small amounts of other fatty acids such as diethylacetic acid does not materially alter the complex thus produced in that it simply replaces one or more moles of valproic acid with a biologically inactive acid.

The salt is useful in the treatment of epileptic seizures or convulsions, and can conveniently be formulated in solid dosage forms, either alone or in combination with one or more pharmaceutically acceptable diluents in accordance with U.S. Patent No. 3,325,361. Typical diluents or excipients include starch, talcum powder, lubricants, disintegrators, flavoring agents, coloring agents or the like.

When used herein in the treatment of epileptic seizures or convulsions, the compounds and compositions of the present invention should be administered in an amount of from 1 to 100 mg/kg body weight/day, preferably 16 to 64 mg/kg body weight/day of valproic acid.

Having described the basic concepts of the invention, reference is now made to the following examples which are provided by way of illustration, and not by way of limitation, of the practice of the invention.

Example

This example illustrates the preparation of the calcium salt of valproic acid.

One mole of $Ca(OH)_2$ (74.08 g) was dissolved in 100 ml of water in a 500 ml volumetric flask equipped with a stirrer and heating mantle. Four moles of valproic acid (576.84 g) were added and the resulting mixture stirred and heated to boiling.

The water was distilled off over a period of approximately 2 hours, and the residue in the flask solidified. That solid was isolated and dried by dissolving in hexane, drying over anhydrous sodium sulfate, filtering and then evaporating the hexane.

The compound obtained has the structure:

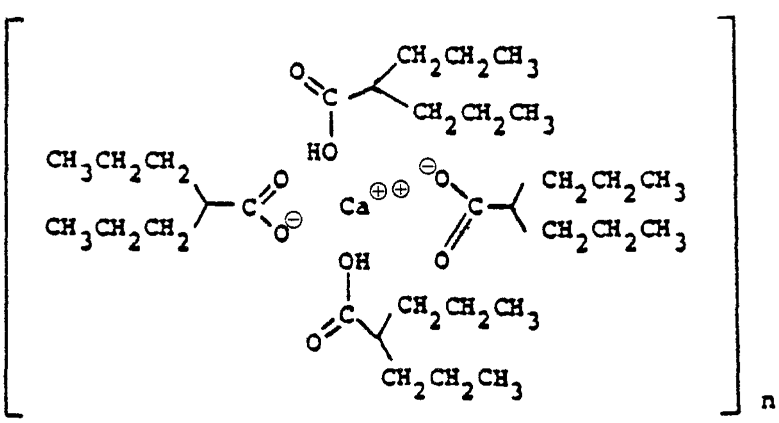

(III)

wherein n is 2 to 20.

It will be understood that various changes and modifications can be made in the details of procedure,

formulation and use without departing from the spirit of the invention, especially as defined in the following claims.

**Claims**

1. A compound of the formula

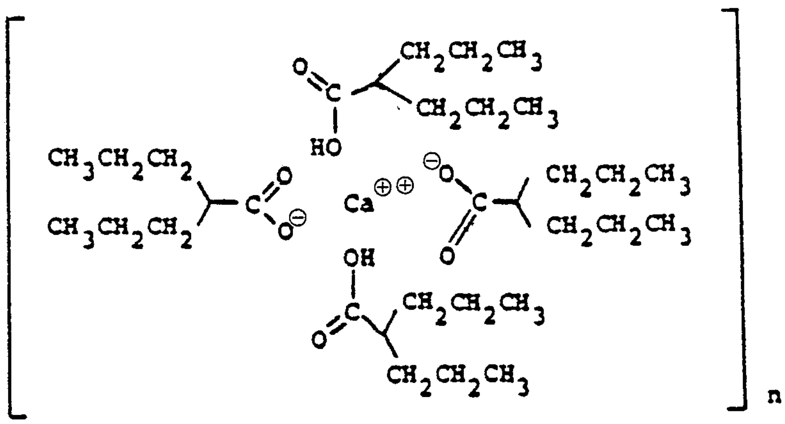

wherein n is 2 to 20.

2. An oral pharmaceutical dosage form containing, as the principal active compound, a compound of the formula:

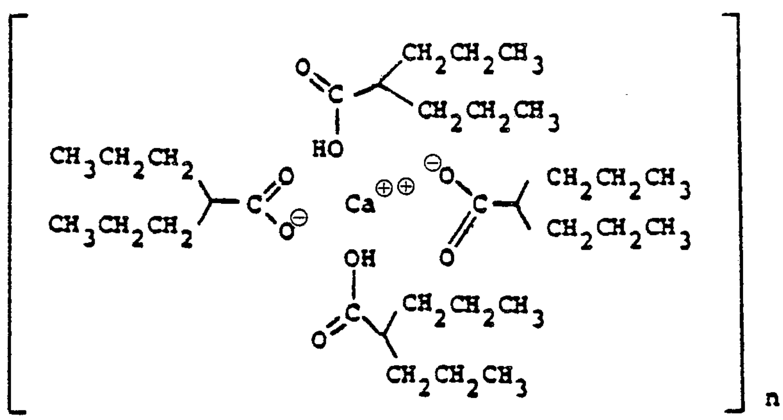

wherein n is 2 to 20, and a pharmaceutically acceptable diluent.

3. Use of a compound of the formula:

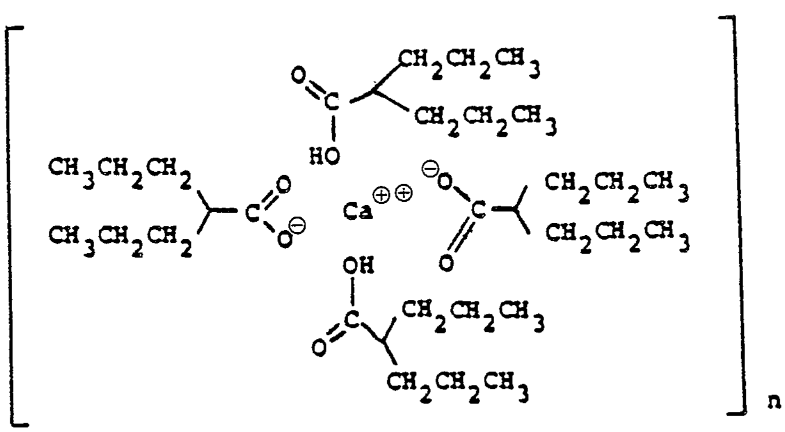

wherein n is 2 to 20, for the preparation of a drug for treating epileptic seizures or convulsions.

**Patentansprüche**

1. Eine Verbindung der Formel

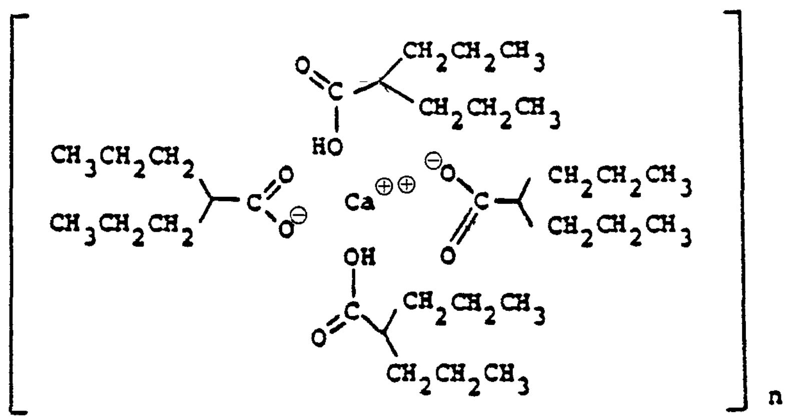

worin n 2 bis 20 ist.

2. Eine orale, pharmazeutische Dosierungsform, enthaltend als die Hauptwirkverbindung eine Verbindung der Formel:

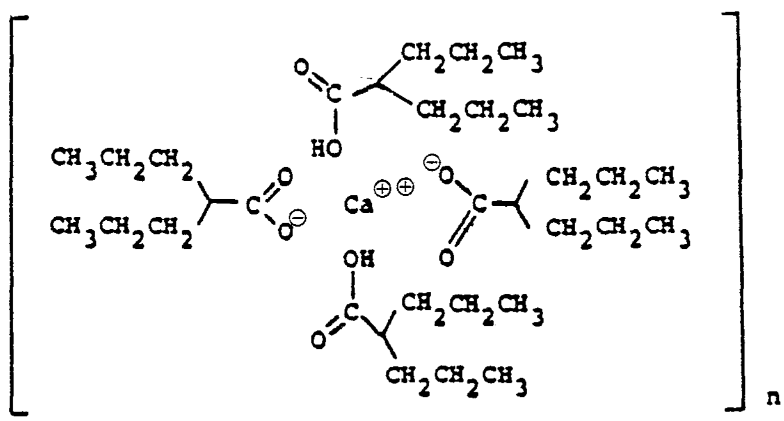

worin n 2 bis 20 ist, und ein pharmazeutisch annehmbares Verdünnungsmittel.

3. Verwendung einer Verbindung der Formel:

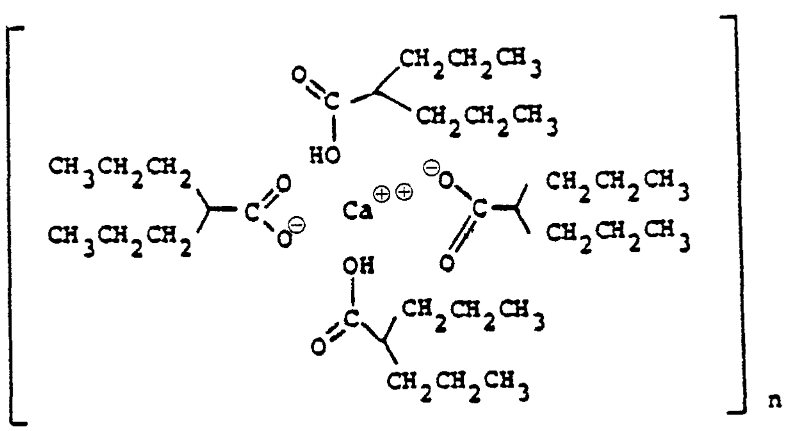

worin n 2 bis 20 ist, zur Herstellung eines Arzneimittels für die Behandlung von epileptischen Anfällen oder Krämpfen.

**Revendications**

1. Un composé de formule

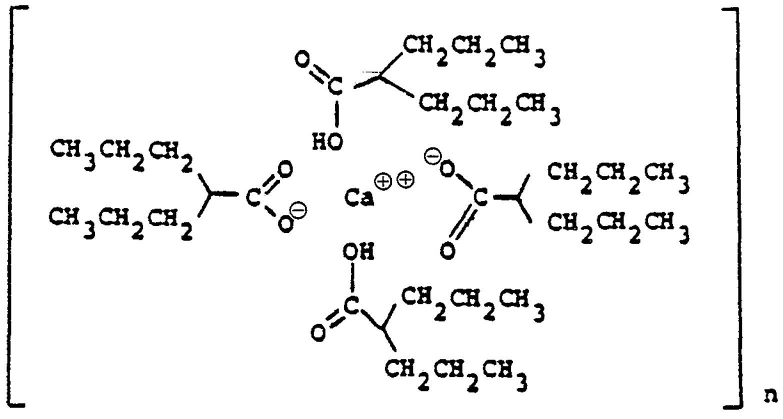

dans laquelle n est de 2 à 20.

2. Une forme de dosage pharmaceutique par voie orale contenant comme composé actif principal un composé de formule:

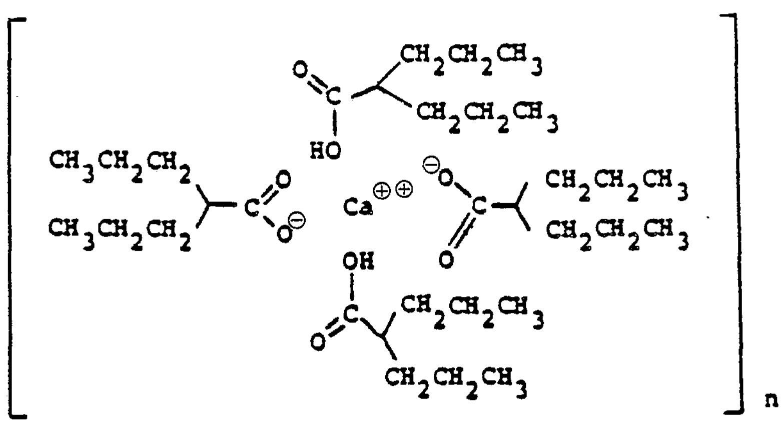

dans laquelle n est de 2 à 20 et un diluant acceptable en pharmacie.

3. Utilisation d'un composé de formule:

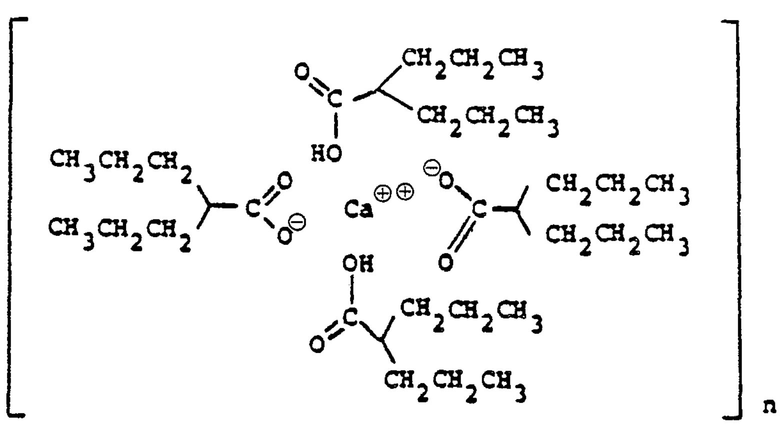

dans laquelle n est de 2 à 20 pour la préparation d'un médicament pour le traitement des crises ou convulsions épileptiques.